# EUROPEAN PATENT APPLICATION

(11) **EP 2 153 806 A1**
(43) Date of publication of application: **17.02.2010**
(21) Application number: 07123126.0
(22) Date of filing: 13.12.2007
(51) Int. Cl.: A61F 11/12, A61F 11/14

(54) **Ear protector**

(71) Applicant: INEOS EUROPE LIMITED, Lyndhurst Hampshire SO43 7FG (GB)
(72) Inventor: The designation of the inventor has not yet been filed
(74) Representative: Smith, Julian Philip Howard

(57) **Abstract**

An ear protector is disclosed comprising an earpiece adapted to protect the ear from excessive noise, and a flexible tube connected at one end to the earpiece, and at its other end to a sound-collecting device which comprises a cup having a diaphragm across its opening to define a sound chamber.

## Description

The present invention relates generally to ear protectors for shielding the human ear from excessive noise, and more particularly to an ear protector adapted to enable the wearer to hear sounds emanating from a specific speaker, such as that of a radio.

Ear protectors are very well known, and typically come in the form either of a "headphone" shell which encloses the entire ear, or a small foam insert which is placed inside the eardrum, or a moulded earpiece which sits in the entrance to the ear.

One obvious disadvantage of ear protectors is that the wearer has much greater difficulty in hearing communications. In order to address this problem, it is known for ear protectors to be augmented with a telephone receiver for example, and even a microphone as well. However such devices are obviously relatively complex and expensive compared with a simple ear protector, and in in many noisy environments workers already have communication devices such as radios, so adding additional microphones and receivers is not cost-effective. Furthermore, in industrial environments where flammable materials may be present, any electronic device requires ATEX certification for use in such environments, which increases the difficulties of developing and introducing a suitable design. ATEX is the name commonly given to the European framework for controlling explosive atmospheres and the standards of equipment and protective systems used in them.

We have devised an ear protector which can be used in conjunction with existing communication equipment, and which is cheap and simple to produce and use.

Accordingly in a first aspect the present invention provides an ear protector comprising an earpiece adapted to protect the ear from excessive noise, and a flexible tube connected at one end to the earpiece, and at its other end to a sound-collecting device which comprises a cup having a diaphragm across its opening to define a sound chamber.

The sound-collecting device is placed by the wearer of the ear protector over the speaker of the radio or walkie-talkie which the wearer uses to communicate. Sound emitted from the speaker of the radio causes the diaphragm to vibrate, and as in a stethoscope the sound is amplified in the sound chamber and is transmitted along the flexible tube to the earpiece, where it can be heard by the ear.

It is a significant advantage of the invention that the ear protector can be used with existing communication equipment, therefore substantially reducing the cost of the system. It can also be constructed using conventional existing designs of ear protectors; indeed, it is possible simply to adapt existing ear protectors. The absence of any electronic or metal parts also means that there is no requirement for ATEX certification. The invention is also extremely lightweight, and with no moving parts it is physically robust.

Usually the flexible tube is connected directly to the earpiece. However in some embodiments of the invention, it may be attached to a rigid member which is itself attached to the earpiece. In such a case, sound is transmitted into the earpiece via the flexible tube and then the rigid member. The rigid member is usually a tube, but may be an attachment for supporting the earpiece or for providing a suitable connection between the flexible tube and the earpiece.

The earpiece may be of any design which functions as an acoustic ear protector. If it is of a conventional headphone design with a cup which fully encloses the ear, the flexible tube usually passes through the cup so that the end of the tube is exposed in the chamber formed by the cup and the wearer's head. Sound transmitted by the tube is emitted into the chamber, such that the earpiece functions similarly to a conventional acoustic headphone.

Alternatively, the earpiece may be in the form of a generally cylindrical block of soft foam or sponge, which inserts into the ear canal. In this case the flexible tube is typically attached to a rigid member, the other end of which inserts into the earpiece. The rigid member forms a support for the earpiece. Preferably the rigid member is tubular, and more preferably is a hollow tube through which the flexible tube is inserted, such that the flexible tube can contact the earpiece itself directly.

A third common form for the earpiece is a moulded piece which sits in auricle of the ear, blocking the ear canal. In this case the flexible tube is directly attached to the earpiece.

The flexible tube may be made of any material which is flexible, and which is capable of transmitting sound along its length. Therefore it is preferably comprised of a single material, and is more preferably an integral piece without any interfaces or junctions. A favoured material is silicone. Other possible materials include soft PVC and soft rubber.

The sound-collecting device is preferably in the form of a cup having an opening covered by a diaphragm so as to form an enclosed chamber. The diaphragm is inset from the opening of the cup, such that the edge of the cup forms a circumferential flange around it. Thus when the edge is placed against a surface such as the speaker of a radio, the diaphragm is spaced from the surface and is free to vibrate. Preferably the circumferential flange is of a resilient soft material such as silicone, soft PVC or soft rubber so as to damp vibrations when the edge of the cup is placed against a speaker surface.

The cup and diaphragm are usually of a generally semicircular or conical cross-section, with the diaphragm at the widest open end and the flexible tube or an attachment therefor inserted into the narrower rear end. The cup is most conveniently made of a metal such as steel, or a rigid polymer. The diaphragm is usually made of a thin sheet of rigid polymer such as PVC, polyethylene, polypropylene or polystyrene.

In use, the sound-collecting device is placed against the loudspeaker of a radio or walkie-talkie or other aural communication device. Thus it is preferred that the sound-collecting device additionally comprises attachment means such as clips, magnets or an elasticated band, so that it can be fixed onto the loudspeaker.

The invention will be further described with reference to the accompanying drawings, which show the sound-collecting device of the invention, and three different types of earpiece to which the invention has been applied.

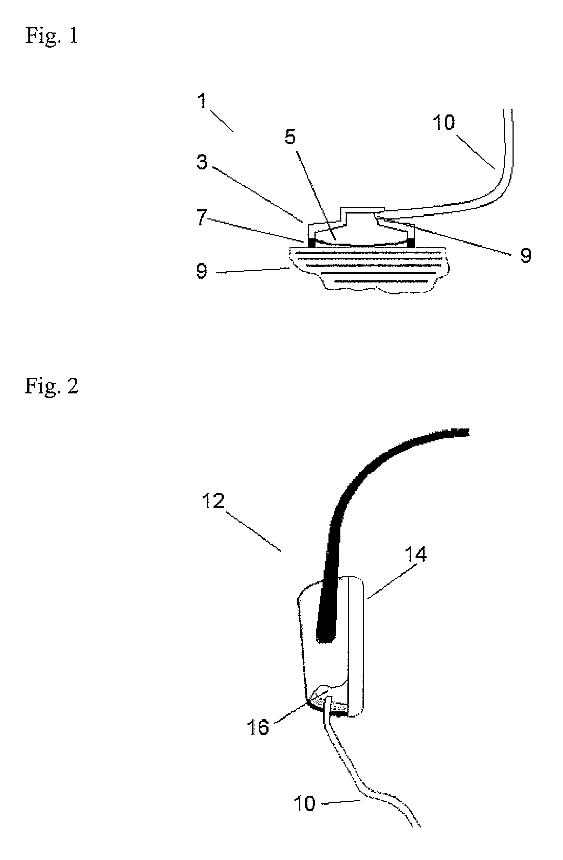

Referring to Figure 1, this shows the sound-collecting device 1, in this case placed on the surface 9 of a loudspeaker, such as that of a walkie-talkie or radio. Device 1 comprises a cup 3 having a diaphragm 5 across its opening so as to define a sound chamber. The diaphragm 5 is spaced from the surface of the loudspeaker 9 by a flange of silicone 7, which acts to damp any vibrations and therefore ensure that the cup is steady. The cup may be secured to the speaker surface by any known means (not shown). In use, sound from the speaker 9 causes diaphragm 5 to vibrate, and these vibrations are amplified in the sound chamber defined by the cup and diaphragm. They are picked up by the projecting end 9 of the flexible tube 10, which is preferably a silicone tube. Tube 10 transmits the vibrations to the earpiece.

Figure 2 shows a first type of earpiece 12. This is a conventional "headphone" type of ear protector, in which the edge 14 of the earpiece completely surrounds the ear. According to the invention, the flexible tube 10 is enters the inner chamber 16 of the earpiece via a hole in the outer wall. Thus sound transmitted along the tube 10 is emitted into the chamber 16, such that the earpiece acts like a conventional acoustic headphone.

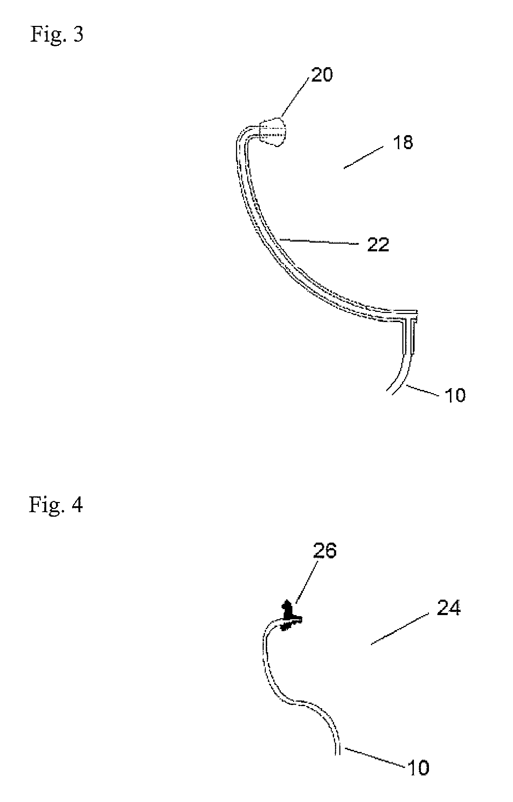

Figure 3 shows another type of earpiece 18, which comprises a soft foam insert 20 which is placed in the entrance of the ear canal. In this case the insert 20 is attached to a rigid tubular piece 22, through which the flexible tube 10 passes so that it contacts the insert 20 directly.

Figure 4 shows a third type of earpiece 24, which simply comprises a moulded piece 26 which sits in the auricle of the ear. In this case, the flexible tube 10 inserts directly into the moulded piece 26.

## Claims

1. Ear protector comprising an earpiece adapted to protect the ear from excessive noise, and a flexible tube connected at one end to the earpiece, and at its other end to a sound-collecting device which comprises a cup having a diaphragm across its opening to define a sound chamber.

2. Ear protector according to claim 1, wherein the earpiece is in the form of a cup which fully encloses the ear, and the flexible tube passes through the cup so that the end of the tube is exposed in the chamber formed by the cup and the wearer's head.

3. Ear protector according to claim 1, wherein the earpiece is in the form of a block of soft foam or sponge for insertion into the ear canal, which block is attached to a rigid member, the member being attached to the flexible tube.

4. Ear protector according to claim 1, wherein the earpiece is in the form of a moulded piece adapted for sitting in auricle of the ear, and the flexible tube is directly attached to the earpiece.

5. Ear protector according to any preceding claim, wherein the flexible tube is formed of a single piece of silicone, soft PVC or soft rubber.

6. Ear protector according to any preceding claim, wherein the sound-collecting device comprises a cup having a diaphragm inset from the opening thereof.

7. Ear protector according to any preceding claim, wherein the end of the flexible tube attached to the cup of the sound-collecting device is exposed inside the sound chamber formed by the cup.
